# EUROPÄISCHE PATENTSCHRIFT

(11) **EP 2 199 240 B1**
(45) Veröffentlichungstag und Bekanntmachung des Hinweises auf die Patenterteilung: **04.04.2012**
(21) Anmeldenummer: 09014256.3
(22) Anmeldetag: 14.11.2009
(51) Int. Cl.: B65H 29/24, B65H 39/14, A61F 13/15, B65G 47/26, B65G 47/91

(54) **Vorrictung zum Vereinzeln von Teilen**
Device for separating parts
Dispositif destiné à la séparation de pièces

(30) Priorität: 18.12.2008 DE 102008063786
(43) Veröffentlichungstag der Anmeldung: 23.06.2010
(73) Patentinhaber: ROMACO Pharmatechnik GmbH, 76227 Karlsruhe (DE)
(72) Erfinder: Konstandin, Horst, 76307 Karlsbad (DE); Herbach, Matthias, 76327 Pfinztal (DE); Kratzmeier, Jürgen, 75015 Bretten (DE); Bippus, Jörg-Uwe, 76275 Ettlingen (DE)
(74) Vertreter: Dimmerling & Huwer

(56) Entgegenhaltungen:
- US-A- 4 200 179
- US-A1- 2005 126 115
- US-A1- 2007 137 982

## Beschreibung

Die Erfindung betrifft eine Vorrichtung nach dem Oberbegriff des Anspruchs 1, zum Vereinzeln von in wenigstens zwei nebeneinander verlaufenden Reihen angeordneten Teilen, mit einem Übergabeelement, welches wenigstens zwei nebeneinander angeordnete Halteelemente aufweist, welche jeweils den vordersten Teil einer Reihe in einer ersten Position aufnehmen und in einer zweiten Position absetzen, wobei Mittel vorhanden sind, mittels welcher der Abstand, in dem die Halteelemente nebeneinander angeordnet sind, beim Transport der Teile von der ersten Position in die zweite Position veränderbar ist, wobei die Mittel zum Verändern des Abstands der Halteelemente als Längs-Führungsschienen ausgebildet sind, mit denen die Halteelemente in Wirkverbindung stehen, wobei mehrere Quer-Führungsschienen vorhanden sind, welche mit unterschiedlichen Geschwindigkeiten entlang der Längs-Führungsschienen verstellbar sind, und wobei die Halteelemente mit wenigstens einer Quer-Führungsschiene in Wirkverbindung stehen.

Eine Vorrichtung zum Vereinzeln von beispielsweise in wenigstens zwei nebeneinander verlaufenden Reihen angeordneten Teilen, mit einem Übergabeelement, welches wenigstens zwei nebeneinander angeordnete Halteelemente aufweist, welche Teile in einer ersten Position aufnehmen und in einer zweiten Position absetzen, ist im Stand der Technik hinlänglich bekannt und ist häufig als um eine Achse drehbares Element ausgebildet, welches mehrere sich in radialer Richtung erstreckende, in einem Abstand nebeneinander und in einem gleichmäßigen Winkelabstand zueinander angeordnete Arme aufweist, an deren Enden sich Vakuumsauger befinden. Mittels der bekannten Vorrichtung lassen sich beispielsweise auf nebeneinander angeordneten Bandfördern angeordnete Teile aufnehmen, um 90 Grad oder 180 Grad schwenken und an ein nachgeordnetes Maschinenelement übergeben.

Die Arme der bekannten Vereinzelungsvorrichtung sind fest an einem drehbaren Element angeordnet, so dass der Abstand, in dem sie nebeneinander angeordnet sind, nicht veränderbar ist. Daher lassen sich die Teile nur in demselben seitlichen Abstand zueinander absetzen, in dem sie aufgenommen wurden. Das heißt es ist mittels der bekannten Vereinzelungsvorrichtung nicht möglich, den Abstand, in dem die Teile nebeneinander angeordnet sind, zu verändern. Dies ist insbesondere dann nachteilig, wenn die Teile unmittelbar, das heißt ohne Abstand, nebeneinander angeordnet sind und es die weitere Bearbeitung erfordert, dass die Teile einen seitlichen Abstand zueinander aufweisen.

Eine Vorrichtung gem. dem Oberbegriff des Anspruchs 1 ist aus US 2005/0126115 A1 bekannt.

Des Weiteren ist aus der US 2007/0137982 A1 eine Vorrichtung bekannt, bei der die Mittel zum Verändern des Abstands als Scherenarme ausgebildet sind.

Es ist Aufgabe der Erfindung, eine Vorrichtung zu schaffen, die es ermöglicht, die Teile zuverlässig von den Halteelementen aufzunehmen.

Die Lösung diese Aufgabe ergibt sich aus den Merkmalen des kennzeichnenden Teils des Anspruchs 1. Vorteilhafte Weiterbildungen der Erfindung ergeben sich aus den Unteransprüchen.

Gemäß der Erfindung ist eine Vorrichtung zum Vereinzeln von in wenigstens zwei nebeneinander verlaufenden Reihen angeordneten Teilen, mit einem Übergabeelement, welches wenigstens zwei nebeneinander angeordnete Halteelemente aufweist, mittels welcher jeweils den vordersten Teil einer Reihe in einer ersten Position aufnehmbar und in einer zweiten Position absetzbar sind, wobei Mittel vorhanden sind, mittels welcher der Abstand, in dem die Halteelemente nebeneinander angeordnet sind, beim Transport der Teile von der ersten Position in die zweite Position veränderbar ist.

Dadurch, dass Mittel vorhanden sind, mittels welcher der Abstand, in dem die Halteelemente nebeneinander angeordnet sind, beim Transport der Teile von der ersten Position in die zweite Position veränderbar ist, lässt sich der seitliche Abstand der Teile zueinander, das heißt der Abstand, in dem die Teile nebeneinander angeordnet sind, auf einfache Weise verändern. Da die Veränderung des Abstands während des Transports der Teile von der ersten Position in die zweite Position erfolgt, ist kein besonderer Maschinenschritt erforderlich, was sich sehr vorteilhaft auf die Zykluszeit der Vereinzelungsvorrichtung auswirkt.

In vorteilhafter Weise sind die Mittel zum Verändern des Abstands der Halteelemente als Längs-Führungsschienen ausgebildet, mit denen die Halteelemente in Wirkverbindung stehen. Hierdurch lässt sich der seitliche Abstand der Halteelemente während des Transports der Teile von der ersten Position in die zweite Position auf einfache Weise verändern. Dies kann dadurch geschehen, dass sich die Längsführungsschienen von der ersten Position bis zur zweiten Position erstrecken, wobei sie in der ersten Position in einem Abstand nebeneinander angeordnet sind, der erforderlich ist, damit die Halteelemente die Teile in der ersten Position aufnehmen können, und in der zweiten Position in einem Abstand nebeneinander angeordnet sind, der erforderlich ist, damit die Halteelemente die Teile in der zweiten Position absetzen können. Die Veränderung des Abstands erfolgt kontinuierlich.

Die Wirkverbindung der Halteelemente mit den Längs-Führungsschienen kann beispielsweise dadurch hergestellt werden, dass die Halteelemente Führungselemente aufweisen, welche in Längsnuten angeordnet sind, die die Längs-Führungsschienen aufweisen. Statt dass die Führungselemente in Längsnuten der Längs-Führungsschienen angeordnet sind, können sie auch zwischen zwei Längs-Führungsschienen angeordnet sein. Hierdurch erübrigt es sich, dass die Längs-Führungsschienen Längsnuten aufweisen, was sich vorteilhaft auf die Herstellungskosten auswirkt. Des Weiteren steht der Bereich der Längs-Führungsschienen, in dem die Längsnuten ausgebildet sein müssten, zur Befestigung der Längs-Führungsschienen zur Verfügung. Die Veränderung des seitlichen Abstands der Halteelemente zueinander lässt sich bei der letztgenannten Ausführungsform dadurch erreichen, dass sich die Breite der Längs-Führungsschienen verändert.

Sehr vorteilhaft ist es, dass die Halteelemente mit wenigstens einer Quer-Führungsschiene in Wirkverbindung stehen, wie dies bei der Erfindung vorgesehen ist. Durch die Quer-Führungsschiene lässt sich der seitliche Abstand der Halteelemente zueinander in zuverlässiger Weise verändern. Die Halteelemente können beispielsweise an Gleitelementen befestigt sein, welche in einer entsprechend ausgebildeten Nut der Quer-Führungsschiene angeordnet sind. Hierdurch sind die Halteelemente zuverlässig verschiebbar befestigt.

Sehr vorteilhaft ist eine Ausführungsform der Erfindung, bei der die wenigstens eine Quer-Führungsschiene um eine gedachte Achse drehbar angeordnet ist. Das heißt, die Vorrichtung ist zylinderförmig ausgebildet. Dies wirkt sich sehr vorteilhaft auf den Raumbedarf der Vorrichtung aus.

Dadurch, dass die Quer-Führungsschiene um eine gedachte Achse drehbar angeordnet ist, müssen die Längs-Führungsschienen einen entsprechenden Verlauf haben. Das heißt, die Längs-Führungsschienen müssen ringförmig ausgebildet sein. Die Halteelemente bewegen sich dann auf einer ringförmigen Bahn, wodurch es möglich ist, die Teile beim Transport von der ersten Position in die zweite Position auf einer Kreisbahn um beispielsweise 180 Grad zu bewegen. Die Teile können beispielsweise von den Halteelementen in senkrechter Position oben aufgenommen werden und in senkrechter Position unten abgegeben werden.

Erfindungsgemäss sind mehrere Quer-Führungsschienen vorhanden, welche mit unterschiedlichen Geschwindigkeiten entlang der Längs-Führungsschienen verstellbar sind. Hierdurch lassen sich gleichzeitig mehrere hintereinander angeordnete Teile transportieren. Dies wirkt sich sehr vorteilhaft auf die Zykluszeit der Vorrichtung aus.

So können beispielsweise bei vier Quer-Führungsschienen Teile gleichzeitig von den Halteelementen aufgenommen und abgesetzt werden, wobei während des Aufnehmens beziehungsweise des Absetzens der Teile die Quer-Führungsschiene, welche zuvor Teile aufgenommen hatte, von der ersten Position in die zweite Position verstellt werden kann sowie die Quer-Führungsschiene, welche zuvor Teile abgesetzt hatte, von der zweiten Position in die erste Position verstellt werden kann. Besonders vorteilhaft ist es hierbei, dass die Halteelemente beim Aufnehmen sowie beim Absetzen der Teile an die Geschwindigkeit der Elemente angepasst werden können, von denen die Teile aufgenommen beziehungsweise auf die die Teile abgesetzt werden sollen. Die Elemente können daher kontinuierlich bewegt werden, wobei die Teile bei der Aufnahme keinen Abstand zueinander aufweisen müssen.

Besonders vorteilhaft bei einer Ausführungsform ist es, wenn die Quer-Führungsschienen an Hohlwellen befestigt sind. Hierdurch ist eine sehr kompakte Bauweise der Vorrichtung möglich.

Bei einer weiteren besonderen Ausführungsform der Erfindung ist vorgesehen, dass die Halteelemente mit Teleskop-Vakuumleitungen verbunden sind, mittels welcher die Halteelemente mit Vakuum beaufschlagbar sind. Durch die Teleskop-Vakuumleitungen lässt sich auf einfache Weise und zuverlässig bei den Halteelementen ein Vakuum erzeugen. Hierdurch lassen sich die Teile sehr schnell und zuverlässig aber dennoch behutsam von den Halteelementen aufnehmen.

Erfindungsgemäss ist im Bereich der ersten Position ein Umlenkelement angeordnet, um welches eine Trägerfolie, auf der die Teile angeordnet sind, umlenkbar ist. Durch das Umlenkelement können die Teile haftend auf der Trägerfolie angeordnet sein. Denn durch das Umlenken der Trägerfolie um das Umlenkelement wird erreicht, dass Teile, welche haftend auf der Trägerfolie angeordnet sind, von der Trägerfolie gelöst werden und zuverlässig von den Halteelemente aufgenommen werden können.

Als sehr vorteilhaft hat sich heraus gestellt, die erfindungsgemäße Vereinzelungsvorrichtung in einer Verpackungsmaschine anzuordnen, mittels der die Teile zwischen zwei Folien anordenbar sind. Insbesondere bei einer Verpackungsmaschine, welche so genannte Mundpflegestreifen (Oral Tabs) zwischen zwei Folien einschweißt, ist die erfindungsgemäße Vereinzelungsvorrichtung sehr gut verwendbar.

Weitere Einzelheiten, Merkmale und Vorteile der vorliegenden Erfindung ergeben sich aus der nachfolgenden Beschreibung eines besonderen Ausführungsbeispiels unter Bezugnahme auf die Zeichnungen.

Es zeigt:
- Fig. 1: eine schematische Darstellung einer erfindungsgemäßen Vereinze- lungsvorrichtung perspektivischer Ansicht,
- Fig. 2: die in Figur 1 dargestellte Vereinzelungsvorrichtung unter Weglassung einiger Elemente,
- Fig. 3: die in Figur 1 dargestellte Vorrichtung unter Weglassung weiterer Elemente und
- Fig. 4: eine schematische Darstellung einer Verpackungsmaschine mit einer erfindungsgemäßen Vereinzelungsvorrichtung.

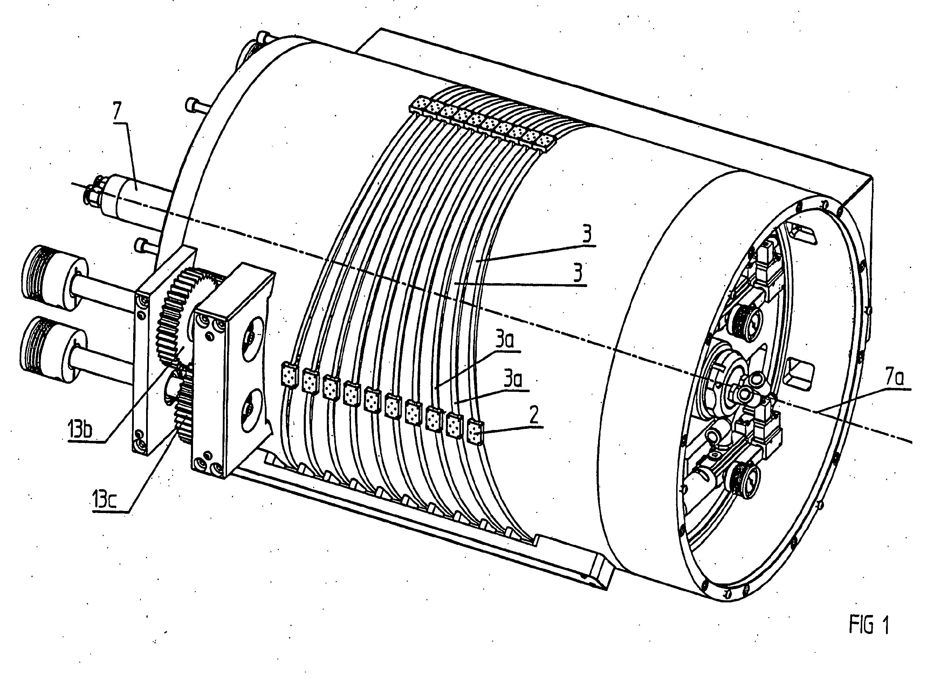

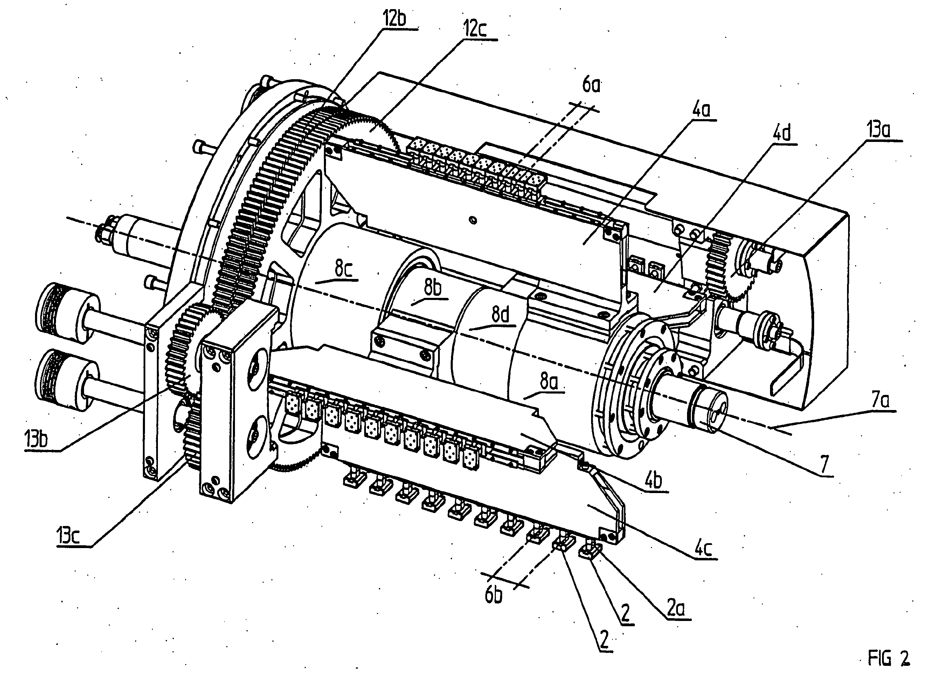

Wie insbesondere den Figuren 1 bis 3 entnommen werden kann, weist eine erfindungsgemäße Vereinzelungsvorrichtung als Vakuumsauger 2 ausgebildete Halteelemente auf, wobei im vorliegenden Fall jeweils zehn Vakuumsauger 2 nebeneinander angeordnet sind. Die Vakuumsauger 2 sind mittels Schlitten, welche über Hälse 2a mit den Vakuumsaugem 2 verbunden sind und jeweils in einer Nut einer Quer-Führungsschiene 4a, 4d verschiebbar angeordnet sind, mit den Quer-Führungsschienen 4a bis 4d verbunden. Dieses lässt sich insbesondere Figur 2 sehr gut entnehmen. Durch die verschiebbare Anordnung der Vakuumsauger 2 an den Quer-Führungsschienen 4a bis 4d lässt sich der Abstand 6a, 6b, in dem die Vakuumsauger 2 nebeneinander angeordnet sind beziehungsweise der Abstand 6a, 6b der Mittellinien der Vakuumsauger 2, verändern.

Die Quer-Führungsschienen 4a bis 4d sind jeweils an einer Hohlwelle 8a bis 8d befestigt, welche auf einer Welle 7 angeordnet sind, welche um eine gedachte Achse 7a drehbar ist. Die Vakuumsauger 2 lassen sich daher auf einer Kreisbahn bewegen. Da jede der Quer-Führungsschienen 4a bis 4d an einer separaten Hohlwelle 8a bis 8d befestigt ist, lassen sich die Quer-Führungsschienen 4a bis 4d und damit die jeweils an einer Quer-Führungsschiene 4a bis 4d angeordneten Vakuumsauger 2 unabhängig voneinander verstellen. Zur Verstellung der Hohlwellen 8a bis 8d sind diese mit Zahnrädern 12a bis 12d verbunden, welche jeweils über Getrieberäder 13a bis 13d von einem in den Figuren nicht dargestellten separaten Antrieb antreibbar sind. Hierdurch können die Vakuumsauger 2 an die Geschwindigkeit von Teilen 1 angepasst werden, die von den Vakuumsaugern 2 aufgenommen werden sollen. Ebenso können die von den Vakuumsaugern 2 aufgenommenen Teile 1 an die Geschwindigkeit einer Bodenfolie 19 angepasst werden, auf die die Teile 1 abgelegt werden sollen. Es ist somit nicht erforderlich, die Teile 1 diskontinuierlich zuzuführen beziehungsweise abzuholen.

Wie insbesondere Figur 1 entnommen werden kann, sind auf der Kreisbahn, auf der die Hälse 2a der Vakuumsauger 2 bewegbar sind, Längs-Führungsschienen 3 angeordnet. Die Längs-Führungsschienen 3 sind in einem Abstand zueinander angeordnet, wodurch zwischen zwei Längs-Führungsschienen 3 ein Schlitz 3a vorhanden ist. Die Breite des Schlitzes 3a entspricht etwa dem Durchmesser der Hälse 2a der Vakuumsauger 2. Die Längs-Führungsschienen 3 sowie die Vakuumsauger 2 sind so ausgebildet, beziehungsweise angeordnet, dass sich die Hälse 2a der Vakuumsauger 2 in den Schlitzen 3a befinden.

Die Breite der Längs-Führungsschienen 3 ist nicht konstant, sondern verändert sich kontinuierlich von einer minimalen Breite senkrecht oben auf der Kreisbahn und einer maximalen Breite senkrecht unten auf der Kreisbahn. Hierdurch verändert sich der Abstand der durch zwei nebeneinander angeordneten Längs-Führungsschienen 3 gebildeten Schlitze 3a kontinuierlich. Da sich die Hälse 2a der Vakuumsauger 2 in den Schlitzen 3a befinden, verändert sich somit auch der Abstand der Vakuumsauger 2 zueinander kontinuierlich, wenn sich die Vakuumsauger 2 auf ihrer Kreisbahn bewegen, das heißt sich die betreffende Quer-Führungsschiene 4 um die gedachte Achse 7a dreht.

Die Längs-Führungsschienen 3 sind so ausgebildet, dass die Vakuumsauger 2 senkrecht oben auf der Kreisbahn nahezu ohne Abstand zueinander nebeneinander angeordnet sind beziehungsweise die Mittellinien der Vakuumsauger 2 in einem ersten Abstand 6a zueinander angeordnet sind. Um 180 Grad versetzt, dass heißt senkrecht unten auf der Kreisbahn, weisen die Vakuumsauger 2 einen Abstand auf, der durch den Abstand vorgegeben ist, in dem die von den Vakuumsaugem 2 aufgenommenen Teile 1 abgesetzt werden sollen. Das heißt, die Mittellinien der Vakuumsauger 2 befinden sich in einem zweiten Abstand 6b zueinander.

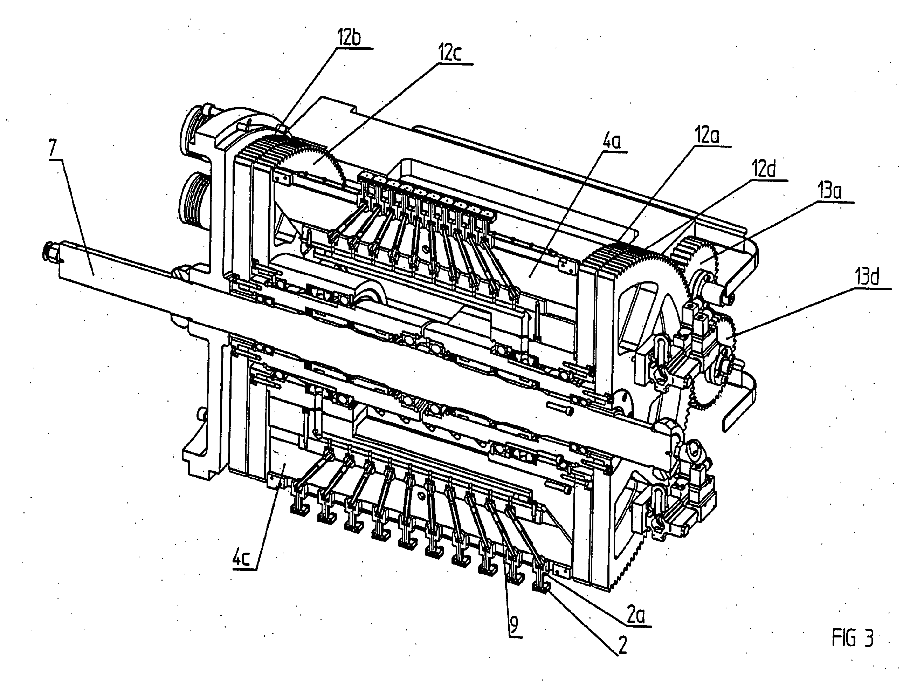

Zur Aufnahme der Teile 1 werden die Vakuumsauger 2 mit einem Vakuum beaufschlagt. Hierzu sind die Vakuumsauger 2 über Teleskop-Vakuumleitungen 9 mit einem Kanal verbunden, in welchem ein Vakuum erzeugbar ist. Dies lässt sich insbesondere der Figur 3 entnehmen.

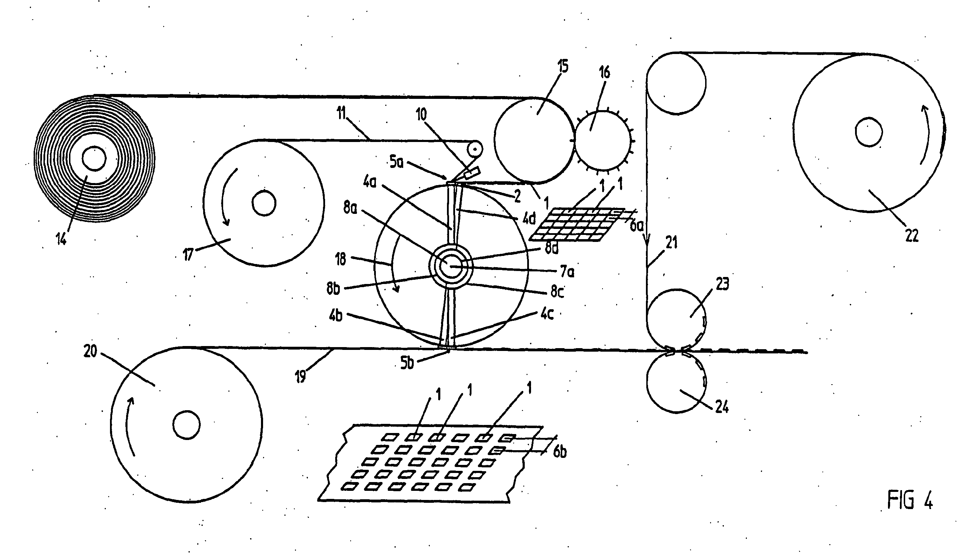

Die Funktionsweise der erfindungsgemäßen Vereinzelungsvorrichtung wird nachfolgenden an Hand der in Figur 4 dargestellten Verpackungsmaschine beschrieben.

Wie Figur 4 entnommen werden kann, ist auf einer Produktrolle 14 ein Trägerband 11, auf welchem auf einer Seite ein Wirkstoff in Form eines Films haftend aufgebracht ist, aufgewickelt. Die Trägerfolie 11 mit ihrem Wirkstofffilm wird durch ein Schneidwalzenpaar 15, 16 geführt, mittels welchem der Wirkstoff in rechteckförmige Teile 1 zerteilt wird. Nach Durchlaufen des Walzenpaares 15, 16 befinden sich auf der Trägerfolie 11 somit einzelne rechteckförmige Teile 1 des filmförmigen Wirkstoffs. Die Teile 1 sind nur durch eine Schnittfuge getrennt, so dass sie im Prinzip unmittelbar aneinander stoßen.

Nach Durchlaufen des Schneidwalzenpaares 15, 16 wird die Trägerfolie 11 um ein als Keil 10 ausgebildetes Umlenkelement geführt, wodurch sich die auf der Trägerfolie 11 haftenden Teile 1 des Wirkstofffilms lösen.

Unterhalb des Keils 10 ist eine erfindungsgemäße Vereinzelungsvorrichtung angeordnet, wobei der Keil 10 beziehungsweise die Vereinzelungsvorrichtung derart angeordnet sind, dass ein Teil 1, nachdem es durch die Umlenkung der Trägerfolie 11 um den Keil 10 von der Trägerfolie 11 gelöst wurde, unmittelbar von einem Vakuumsauger 2 aufgenommen werden kann. Hierzu müssen sich die betreffenden Vakuumsauger 2 beziehungsweise die betreffende erste Quer-Führungsschiene 4a in einer ersten Position 5a befinden. Bei einem kontinuierlichen Transport der Trägerfolie 11 wird die betreffende Hohlwelle 8a der betreffenden Quer-Führungsschiene 4a mittels ihres Antriebs mit einer solchen Geschwindigkeit angetrieben, dass sich die betreffenden Vakuumsauger 2 mit derselben Geschwindigkeit bewegen, wie die Trägerfolie 11. Die Trägerfolie 11 wird nach der Umlenkung um den Keil 10 auf eine Folienrolle 17 aufgewickelt.

Die Vereinzelungsvorrichtung weist vier Quer-Führungsschienen 4a bis 4d auf. Die Anzahl der an den Quer-Führungsschienen 4a bis 4d jeweils angeordneten Vakuumsauger 2 entspricht der Anzahl der auf der Trägerfolie 11 nebeneinander angeordneten Teile 1. Somit können gleichzeitig alle auf der Trägerfolie 11 nebeneinander angeordnete Teile 1 aufgenommen werden.

In Figur 4 ist die erste Quer-Führungsschiene 4a in einer Position dargestellt, in der sich an ihr angeordneten Vakuumsauger 2 in ihrer erste Position 5a befinden. Nachdem die Teile 1 von den betreffenden Vakuumsaugern 2 aufgenommen wurden, wird die betreffende Quer-Führungsschiene 4a mit großer Geschwindigkeit in Richtung des Pfeils 18 bewegt, bis sie sich die Vakuumsauger 2 vor einer zweiten Position 5b befinden. In dieser Position ist in Figur 4 die zweite Quer-Führungsschiene 4b dargestellt. In der zweiten Position 5b, in der in Figur 4 die dritte Quer-Führungsschiene 4c dargestellt ist, werden die Teile 1 auf die bereits erwähnte Bodenfolie 19, welche von einer Bodenfolienrolle 20 abgewickelt wird, abgelegt.

Während die an der ersten Quer-Führungsschiene 4a angeordneten Vakuumsauger 2 Teile 1 von der Trägerfolie 11 abnehmen, werden gleichzeitig von den Vakuumsaugern 2 der sich bereits in der zweiten Position 5b befindlichen dritten Quer-Führungsschiene 4c Teile 1 auf die Bodenfolie 19 abgelegt.

Da die Teile 1 auf der Bodenfolie 19 hintereinander in einem Abstand zueinander abgelegt werden, bewegt sich die Bodenfolie 19 in einer größeren Geschwindigkeit als die Trägerfolie 11. Bei einem kontinuierlichen Transport der Bodenfolie 19 wird die betreffende Hohlwelle 8c der betreffenden dritten Quer-Führungsschiene 4c mittels ihres Antriebs mit einer solchen Geschwindigkeit angetrieben, dass sich die betreffenden Vakuumsauger 2 mit derselben Geschwindigkeit bewegen, wie die Bodenfolie 19.

Nachdem die Teile 1 von den betreffenden Vakuumsaugern 2 auf die Bodenfolie 19 abgelegt wurden, wird die betreffende Quer-Führungsschiene 4c mit großer Geschwindigkeit in Richtung des Pfeils 18 bewegt, bis sich die an ihr angeordneten Vakuumsauger 2 kurz vor der ersten Position 5a befinden, in der die Teile 1 von den betreffenden Vakuumsaugern 2 von der Trägerfolie 11 aufgenommen werden. In dieser Position ist in Figur 4 die vierte Quer-Führungsschiene 4d dargestellt.

Da sich die Hälse 2a der Vakuumsauger 2 in den Schlitzen 3a befinden, und sich der Abstand der Schlitze 3a zueinander von der ersten Position 5a bis zur zweiten Position 5b verändert, verändert sich auf dem Weg der Vakuumsauger 2 von der ersten Position 5a in die zweite Position 5b auch der Abstand, in dem die betreffenden Vakuumsauger 2 nebeneinander angeordnet sind, beziehungsweise der Abstand 6a, 6bder Mittellinien der betreffenden Vakuumsaugern 2. Die Teile 1 weisen daher in der zweiten Position 5b einen seitlichen Abstand 6b zueinander auf, der größer ist, als ihr seitlicher Abstand 6a in der ersten Position 5a. Der seitliche Abstand 6b in der zweiten Position 5b entspricht dem Abstand in dem die Teile 1 auf die Bodenfolie 19 abgelegt werden.

In gleicher Weise, in der sich der Abstand der Vakuumsauger auf dem Weg von der ersten Position 5a in die zweite Position 5b verändert, verändert sich der seitliche Abstand der Vakuumsauger 2 zueinander auf dem Weg von der zweiten Position 5b in die erste Position 5a. Das heißt, nachdem die Vakuumsauger 2 von der zweiten Position 5b in die erste Position 5a verstellt wurden, weisen ihre Mittellinien nicht mehr den zweiten Abstand 6b auf, den sie in der zweiten Position 5b hatten, sondern den geringeren ersten Abstand 6a auf, was heißt, dass die Vakuumsauger unmittelbar nebeneinander angeordnet sein können.

Nachdem die Teile 1 auf der Bodenfolie 19 abgelegt wurden, wird zur Abdeckung der Teile 1 auf die Bodenfolie 19 sowie auf die Teile 1 eine Deckfolie 21, welche von einer Deckfolienrolle 22 abgewickelt wird, aufgelegt. Nachdem die Deckfolie 21 auf die Bodenfolie 19 aufgelegt wurde, durchläuft die aus Bodenfolie 19, Teile 1 und Deckfolie 21 bestehende Anordnung ein Siegelwalzenpaar 23, 24, mittels den die Deckfolie 21 mit der Bodenfolie 19 verschweißt wird.

## Patentansprüche

1. Vorrichtung zum Vereinzeln von in wenigstens zwei nebeneinander verlaufenden Reihen angeordneten Teilen (1), mit einem Übergabeelement (2, 4), welches wenigstens zwei nebeneinander angeordnete Halteelemente (2) aufweist, welche jeweils den vordersten Teil einer Reihe in einer ersten Position (5a) aufnehmen und in einer zweiten Position (5b) absetzen, wobei Mittel (3) vorhanden sind, mittels welcher der Abstand (6a, 6b), in dem die Halteelemente (2) nebeneinander angeordnet sind, beim Transport der Teile (1) von der ersten Position (5a) in die zweite Position (5b) veränderbar ist, wobei die Mittel (3) zum Verändern des Abstands (6a, 6b) der Haltelemente (2) als Längs-Führungsschienen (3) ausgebildet sind, mit denen die Halteelemente (2) in Wirkverbindung stehen, wobei mehrere Quer-Führungsschienen (4a bis 4d) vorhanden sind, welche mit unterschiedlichen Geschwindigkeiten entlang der Längs-Führungsschienen (3) verstellbar sind, und wobei die Halteelemente (2) mit wenigstens einer Quer-Führungsschiene (4a bis 4d) in Wirkverbindung stehen,
**dadurch gekennzeichnet,**
**dass** im Bereich der ersten Position (5a) ein Umlenkelement (10) angeordnet ist, um welches eine Trägerfolie (11), auf der die Teile (1) angeordnet sind, umlenkbar ist.

2. Vorrichtung nach Anspruch 1,
**dadurch gekennzeichnet,**
**dass** die wenigstens eine Quer-Führungsschiene (4a bis 4d) um eine gedachte Achse (7a) drehbar angeordnet ist.

3. Vorrichtung nach Anspruch 1 oder 2,
**dadurch gekennzeichnet,**
**dass** die Quer-Führungsschienen (4a bis 4d) an Hohlwellen (8a bis 8d) befestigt sind.

4. Vorrichtung nach einem der Ansprüche 1 bis 3,
**dadurch gekennzeichnet,**
**dass** die Halteelemente (2) mit Teleskop-Vakuumleitungen (9) verbunden sind, mittels welcher die Halteelemente (2) mit Vakuum beaufschlagbar sind.

5. Verpackungsmaschine,
**dadurch gekennzeichnet,**
**dass** sie eine Vorrichtung nach einem der Ansprüche 1 bis 4 aufweist.

## Claims

1. Device for separating parts (1) arranged in at least two adjacent rows, comprising a transfer element (2, 4) which is provided with at least two holding elements (2) which are arranged adjacent to one another and which each pick up the frontmost part of a row in a first position (5a) and deposit said part in a second position (5b), and means (3) are provided by which the distance (6a, 6b) at which the holding elements (2) are arranged adjacent to one another is changeable during the transport of the parts (1) from the first position (5a) to the second position (5b), the means (3) for adjusting the distance (6a, 6b) of the holding elements (2) are configured as longitudinal guide rails (3) to which the holding elements (2) are operatively connected, wherein a plurality of transverse guide rails (4a to 4d) which are adjustable with different speeds along the longitudinal guide rails (3) are provided, and wherein the holding elements (2) are operatively connected to at least one transverse guide rail (4a to 4d), **characterised in that** arranged in the region of the first position (5a) is a deflection element (10) round which a carrier film (11) on which the parts (1) are arranged can be deflected.

2. Device according to claim 1, **characterised in that** the at least one transverse guide rail (4a to 4d) is arranged rotatable about an imaginary axis (7a).

3. Device according to claim 1 or 2, **characterised in that** the transverse guide rails (4a to 4d) are fastened to hollow shafts (8a to 8d).

4. Device according to one of the claims 1 to 3, **characterised in that** the holding elements (2) are connected to telescopic vacuum lines (9) by means of which a vacuum can be applied to the holding elements (2).

5. Packaging machine, **characterised in that** said machine comprises a device according to one of the claims 1 to 4.

## Revendications

1. Dispositif conçu pour la dissociation de pièces (1) agencées en au moins deux rangées s'étendant en juxtaposition, équipé d'un élément de transfert (2, 4) comportant au moins deux éléments de retenue (2) qui sont placés en juxtaposition, reçoivent respectivement la partie la plus avancée d'une rangée, en un premier emplacement (5a), puis la déposent en un second emplacement (5b), des moyens (3) étant présents pour permettre de faire varier, au cours du transport desdites pièces (1) dudit premier emplacement (5a) audit second emplacement (5b), l'espacement (6a, 6b) selon lequel les éléments de retenue (2) sont agencés en juxtaposition, sachant que, pour faire varier ledit espacement (6a, 6b) des éléments de retenue (2), lesdits moyens (3) sont réalisés sous la forme de rails (3) de guidage longitudinal avec lesquels lesdits éléments de retenue (2) sont en liaison opérante, plusieurs rails (4a à 4d) de guidage transversal pouvant être déplacés à des vitesses différentes le long desdits rails (3) de guidage longitudinal, et lesdits éléments de retenue (2) étant en liaison opérante avec au moins un rail (4a à 4d) de guidage transversal,
**caractérisé par le fait**
**qu'**un élément de renvoi (10), autour duquel peut être renvoyé un substrat pelliculaire (11) sur lequel les pièces (1) sont disposées, se trouve dans la région du premier emplacement (5a).

2. Dispositif selon la revendication 1,
**caractérisé par le fait**
**que** le rail (4a à 4d) de guidage transversal, à présence minimale, est monté à rotation autour d'un axe imaginaire (7a).

3. Dispositif selon la revendication 1 ou 2,
**caractérisé par le fait**
**que** les rails (4a à 4d) de guidage transversal sont fixés à des arbres creux (8a à 8d).

4. Dispositif selon l'une des revendications 1 à 3,
**caractérisé par le fait**
**que** les éléments de retenue (2) sont reliés à des conduits télescopiques de dépression (9), au moyen desquels lesdits éléments de retenue (2) peuvent être sollicités par un vide.

5. Machine d'emballage
**caractérisée par le fait**
**qu'**elle présente un dispositif conforme à l'une des revendications 1 à 4.
